(19) Europäisches Patentamt
European Patent Office
Office européen des brevets

(11) Veröffentlichungsnummer: **0 276 721**
**A1**

(12) # EUROPÄISCHE PATENTANMELDUNG

(21) Anmeldenummer: **88100645.6**

(22) Anmeldetag: **19.01.88**

(51) Int. Cl.⁴: **C07C 131/00**, C07C 147/02, C07C 147/14, C07C 149/20, C07C 121/43, C07D 207/02, C07D 211/84

(30) Priorität: **27.01.87 DE 3702282**

(43) Veröffentlichungstag der Anmeldung:
**03.08.88 Patentblatt 88/31**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Lunkenheimer, Winfried, Dr.**
**Bismarckstrasse 29**
**D-5600 Wuppertal 1(DE)**

(54) **2-Cyan-2-oximino-acetamide.**

(57) 2-Cyan-2-oximino-acetamide der allgemeinen Formel (I)

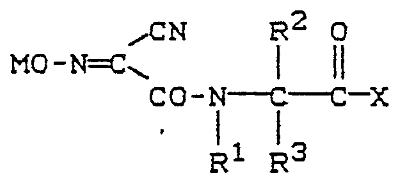

(I)

in welcher
M, $R^1$ bis $R^3$ und X die in der Beschreibung gegebene Bedeutung haben, sowie ihre Verwendung als Ausgangsstoffe für hochaktive Folgeverbindungen. Neue Zwischenprodukte zur Herstellung der Verbindungen der Formel (I).

Die neuen Verbindungen der Formel (I) können z.B. aus geeigneten Cyanacetylaminen mit salpetriger Säure oder Derivaten davon hergestellt werden.

EP 0 276 721 A1

## 2-Cyan-2-oximino-acetamide

Die vorliegende Erfindung betrifft neue 2-Cyan-2-oximino-acetamide, ein Verfahren zu deren Herstellung und ihre Verwendung als Zwischenprodukte zur Synthese z.B. von N-(2-Cyan-2-alkoximinoacetyl)-aminosäurederivaten, welche u.a. fungizide Eigenschaften besitzen.

Es ist bereits bekannt, daß bestimmte 2-Cyan-2-oximino-acetamide und deren Verwendung als Fungizide bekannt sind (vgl. EP-OS 0 201 999; nicht vorveröffentlichte deutsche Patentanmeldungen P 3 521 131.8 vom 13.06.1985 und P 3 602 243.8 vom 25.01.1986).

Es wurden neue 2-Cyan-2-oximino-acetamide der allgemeinen Formel (I)

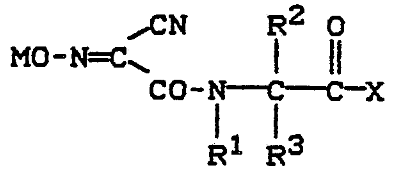

(I)

in welcher

M für Wasserstoff, ein Alkalimetallion, eine protonierte tertiäre Base oder ein quaternäres Ammoniumion steht;

$R^1$ für Wasserstoff, Alkyl, oder jeweils gegebenenfalls substituiertes Phenyl oder Benzyl steht;

$R^2$ für Wasserstoff oder Alkyl steht;

$R^3$für Wasserstoff, Alkyl, Alkoxycarbonylalkyl, Hydroxycarbonylalkyl, Aminocarbonylalkyl, Azolylalkyl, Cyanalkyl, Hydroxyalkyl, Alkenyl, Alkinyl, gegebenenfalls substituiertes Cycloalkyl, für gegebenenfalls substituiertes Phenyl oder Phenylalkyl oder für die Gruppierung

$R^4$-$SO_n$-Z-steht, wobei

$R^4$ für Wasserstoff, Alkyl oder gegebenenfalls substituiertes Phenylalkyl steht;

n für die Zahlen 0, 1 oder 2 steht und

Z für eine geradkettige oder verzweigte Alkylenkette steht, oder

$R^1$ und $R^3$ gemeinsam mit dem Stickstoff-Atom und dem Kohlenstoffatom, an die sie gebunden sind, für einen 5-oder 6-gliedrigen Heterocyclus stehen, oder

$R^2$ und $R^3$ gemeinsam mit dem Kohlenstoffatom, an das sie gebunden sind, für Cycloalkyliden stehen und

X für die Gruppierung -$OR^I$ oder -$NR^{II}R^{III}$ steht, wobei

$R^I$ für Wasserstoff, Alkenyl oder für Alkinyl steht;

$R^{II}$ für Wasserstoff oder Alkyl steht;

$R^{III}$ für Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl oder gegebenenfalls substituiertes Cycloalkyl steht; oder außerdem auch für Alkyl steht, wenn gleichzeitig $R^{II}$ für Alkyl steht,

oder

$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann,

gefunden.

Die 2-Cyan-2-oximino-acetamide der Formel (I) können als E-Isomere, als Z-Isomere bzw. als E,Z-Isomerengemische vorkommen.

Die Verbindungen der Formel (I) besitzen für den Fall, daß $R^2$ und $R^3$ unterschiedliche Bedeutung haben, ein asymmetri sches Kohlenstoffatom; sie können somit auch als optische Isomere (D-und L-Konfiguration) vorliegen, die in unterschiedlichen Mengenverhältnissen anfallen können. Vorwiegend fallen sie als Racemate an.

Weiterhin wurde gefunden, daß man die neuen 2-Cyan-2-oximino-acetamide der Formel (I)

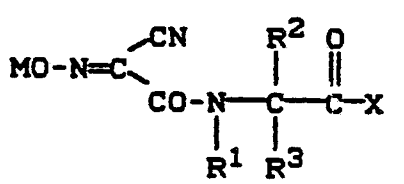

(I)

in welcher

M für Wasserstoff, ein Alkalimetallion, eine protonierte tertiäre Base oder ein quaternäres Ammoniumion steht;

$R^1$ für Wasserstoff, Alkyl, oder jeweils gegebenenfalls substituiertes Phenyl und Benzyl steht;

$R^2$ für Wasserstoff oder Alkyl steht;

$R^3$ für Wasserstoff, Alkyl, Alkoxycarbonylalkyl, Hydroxycarbonylalkyl, Aminocarbonylalkyl, Azolylalkyl, Cyanalkyl, Hydroxyalkyl, Alkenyl, Alkinyl, gegebenenfalls substituiertes Cycloalkyl, für gegebenenfalls substituiertes Phenyl und Phenylalkyl oder für die Gruppierung

$R^4$-$SO_n$-Z-steht, wobei

$R^4$ für Wasserstoff, Alkyl oder gegebenenfalls substituiertes Phenylalkyl steht;

n für die Zahlen 0, 1 oder 2 steht und

Z für eine geradkettige oder verzweigte Alkylenkette steht, oder

$R^1$ und $R^3$ gemeinsam mit dem Stickstoff-Atom und dem Kohlenstoffatom, an die sie gebunden sind, für einen 5-oder 6-gliedrigen Heterocyclus stehen, oder

$R^2$ und $R^3$ gemeinsam mit dem Kohlenstoffatom, an das sie gebunden sind, für Cycloalkyliden stehen und

X für die Gruppierung -$OR^I$ oder -$NR^{II}R^{III}$ steht, wobei

$R^I$ für Wasserstoff, Alkenyl oder für Alkinyl steht;

$R^{II}$ für Wasserstoff oder Alkyl steht;

$R^{III}$ für Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl oder gegebenenfalls substituiertes Cycloalkyl steht;

oder außerdem auch für Alkyl steht, wenn gleichzeitig $R^{II}$ für Alkyl steht,

oder

$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann,

erhält, wenn man z.B. Verbindungen der Formel (II)

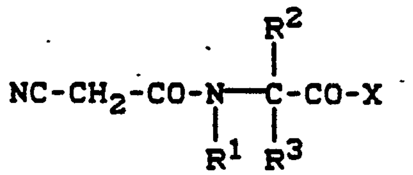

(II)

in welcher

$R^1$, $R^2$, $R^3$ und X die oben angegebene Bedeutung haben,

mit salpetriger Säure oder Derivaten der salpetrigen Säure, gegebenenfalls in Gegenwart eines Verdünnungsmittels und gegebenenfalls in Gegenwart einer Säure oder einer Base umsetzt.

Die neuen 2-Cyan-2-oximino-acetamide sind interessante Zwischenprodukte zur Herstellung von Pflanzenschutzwirkstoffen. So eignen sich die Verbindungen der Formel (I) z.B. als Ausgangsstoffe zur Synthese von N-(2-Cyan-2-alkoximinoacetyl)-aminosäurederivaten, welche sehr gute fungizide Wirksamkeit besitzen (vgl. z.B. EP-OS 0 201 999 nicht vorveröffentlichte deutsche Patentanmeldungen P 3 521 131.8 vom 13.06.1985 und P 3 602 243.8 vom 25.01.1986).

Überraschenderweise sind die N-(2-Cyan-2-alkoximinoacetyl)-aminosäurederivate, die sich aus den erfindungsgemäßen Verbindungen der Formel (I) durch Umsetzung mit dem entsprechenden Alkylierungsmittel herstellen lassen, der aus dem Stand der Technik bekannten Verbindung Zink-ethylen-1,2-bis-dithiocarbamat bezüglich ihrer fungiziden Wirksamkeit überliegen.

Die erfindungsgemäßen Stoffe sind durch die Formel (I) allgemein definiert. Bevorzugt sind Verbindungen der Formel (I), in denen

M für Wasserstoff oder für ein Kalium-oder Natriumäquivalent steht,

$R^1$ für Wasserstoff, geradkettiges oder verzweigtes Alkyl mit 1 bis 4 Kohlenstoffatome, für jeweils gegebenenfalls einfach bis dreifach, gleich oder verschieden substituiertes Phenyl oder Benzyl steht, wobei als Substituenten vorzugsweise genannt seien: Halogen, Cyano, Nitro, Hydroxy; ferner Alkyl, Alkoxy, Alkylthio, Alkylsulfinyl oder Alkylsulfonyl mit jeweils 1 bis 4 Kohlenstoffatomen und gegebenenfalls einfach bis dreifach, gleich oder verschieden durch Halogen substituiertes Phenyl;

$R^2$ für Wasserstoff oder geradkettiges oder verzweigtes Alkyl mit 1 bis 4 Kohlenstoffatomen steht;

$R^3$ für Wasserstoff, geradkettiges oder verzweigtes Alkyl mit 1 bis 6 Kohlenstoffatomen, für geradkettiges

oder verzweigtes Hydroxyalkyl mit 1 bis 4 Kohlenstoffatomen, für Alkoxycarbonylalkyl mit jeweils 1 bis 4 Kohlenstoffatomen im Alkoxyteil und im Alkylteil, für Hydroxycarbonylalkyl und Aminocarbonylalkyl mit jeweils 1 bis 4 Kohlenstoffatomen im Alkylteil, für 1,2,4-Triazol-1-ylalkyl, 1,2,4-Triazol-4-ylalkyl, Imidazol-4-ylalkyl oder Pyrazol-1-ylalkyl mit jeweils 1 bis 6 Kohlenstoffatomen im Alkylteil, für Cyanalkyl mit 1 bis 4 Kohlenstoffatomen im Alkylteil, für Alkenyl oder Alkinyl mit jeweils 2 bis 4 Kohlenstoffatomen, für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils vorzugsweise die bei $R^1$ bereits genannten Phenylsubstituenten in Frage kommen,

oder für die Gruppierung

$R^4$-$SO_n$-Z-steht, wobei

$R^4$ für Wasserstoff, geradkettiges oder verzweigtes Alkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten die bei $R^1$ bereits genannten Phenylsubstituenten in Frage kommen,

n für die Zahlen 0, 1 oder 2 steht und

Z für eine geradkettige oder verzweigte Alkylenkette mit 1 bis 4 Kohlenstoffatomen steht, oder

$R^1$ und $R^3$ gemeinsam mit dem Stickstoffatom und dem Kohlenstoffatom, an die sie gebunden sind, für einen 5-oder 6-gliedrigen Heterocyclus stehen, oder

$R^2$ und $R^3$ gemeinsam mit dem Kohlenstoffatom, an das sie gebunden sind, für Cycloalkyliden mit 3 bis 6 Kohlenstoffatomen stehen;

X für die Gruppierungen -$OR^I$ oder -$NR^{II}R^{III}$steht, wobei

$R^I$ für Wasserstoff, Alkenyl oder Alkinyl mit jeweils 2 bis 4 Kohlenstoffatomen steht;

$R^{II}$ für Wasserstoff oder geradkettiges oder verzweigtes Alkyl mit 1 bis 4 Kohlenstoffatomen steht;

$R^{III}$ für Alkenyl oder Alkinyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit 1 bis 4 Kohlenstoffatomen in den einzelnen Alkylteilen,für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei $R^1$ bereits genannten Phenylsubstituenten in Frage kommen, für gegebenenfalls gleich oder verschieden substituiertes Cycloalkyl mit 3 bis 7 Kohlenstoffatome, wobei als Substituenten vorzugsweise Halogen und Alkyl mit 1 bis 4 Kohlenstoffatomen in Frage kommen; $R^{III}$steht auch für geradkettiges oder verzweigtes Alkyl mit 1 bis 4 Kohlenstoffatomen, wenn $R^{II}$gleichzeitig für Alkyl steht;

oder

$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff oder Stickstoff als weitere Heteroatome enthalten kann und gegebenenfalls durch Cyano, Halogen, Alkyl mit bis 4 Kohlenstoffatomen, Hydroxycarbonyl, Alkoxycarbonyl mit 1 bis 4 Kohlenstoffatomen im Alkoxyteil, Aminocarbonyl, Alkylaminocarbonyl und Dialkylaminocarbonyl mit jeweils 1 bis 4 Kohlenstoffatomen in den einzelnen Alkylteilen substituiert sein kann.

Besonders bevorzugt sind diejenigen Verbindungen der Formel (I), in denen

M für Wasserstoff, für ein Kalium-oder Natriumäquivalent steht,

$R^1$ für Wasserstoff, Methyl, Ethyl, für jeweils gegebenenfalls einfach oder zweifach, gleich oder verschiedenen substituiertes Phenyl oder Benzyl steht, wobei als Substituenten insbesondere genannt seien: Fluor, Chlor, Cyano, Nitro, Hydroxy, Methyl, Methoxy, Methylthio, Methylsulfinyl, Methylsulfonyl und gegebenenfalls einfach oder zweifach, gleich oder verschieden durch Fluor oder Chlor substituiertes Phenyl steht;

$R^2$ für Wasserstoff, Methyl oder Ethyl steht;

$R^3$ für Wasserstoff, geradkettiges oder verzweigtes Alkyl mit 1 bis 4 Kohlenstoffatomen, Hydroxymethyl, 1-Hydroxyethyl, für Methoxycarbonylmethyl, Methoxycarbonylethyl, Ethoxycarbonylmethyl, Ethoxycarbonylethyl, Hydroxycarbonylmethyl, Hydroxycarbonylethyl, Aminocarbonylmethyl, Aminocarbonylethyl, für 1,2,4-Triazol-1-ylalkyl, 1,2,4-Triazol-4-ylalkyl, Imidazol-4-ylalkyl und Pyrazol-1-ylalkyl mit jeweils 1 bis 4 Kohlenstoffatomen im Alkylteil, für Cyanmethyl, Cyanethyl, für Allyl, für Propargyl, für jeweils gegebenenfalls einfach oder zweifach, gleich oder verschieden durch Fluor, Chlor oder Methyl substituiertes Cyclopropyl,

Cyclobutyl, Cyclopentyl, Cyclohexyl oder Cycloheptyl steht, ferner für gegebenenfalls im Phenylteil einfach oder zweifach, gleich oder verschieden substituiertes Phenylalkyl mit 1 oder 2 Kohlenstoffatomen im Alkylteil oder einfach oder zweifach, gleich oder verschieden substituiertes Phenyl steht, wobei als Substituenten die bei $R^1$ bereits genannten Phenylsubstituenten in Frage kommen, ferner für die Gruppierung

$R^4$-$SO_n$-Z-steht, wobei

$R^4$ für Wasserstoff, Methyl, Ethyl oder für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere die bei $R^1$ bereits genannten Phenylsubstituenten in Frage kommen,

n für die Zahlen 0, 1 oder 2 steht und

Z für eine geradkettige oder verzweigte Alkylenkette mit 1 oder 2 Kohlenstoffatomen steht, oder

$R^1$ und $R^2$ gemeinsam mit dem Stickstoffatom und dem Kohlenstoffatom, an die sie gebunden sind, für einen 5-oder 6-gliedrigen Heterocyclus, wie z.B. Piperidin und Pyrrolidin, stehen, oder

$R^2$ und $R^3$ gemeinsam mit dem Kohlenstoffatom, an das sie gebunden sind, für Cyclopropyliden stehen, und

X für die Gruppierung -$OR^I$ oder -$NR^{II}R^{III}$ steht, wobei

$R^I$ für Wasserstoff, Allyl oder Propargyl steht;

$R^{II}$ für Wasserstoff, Methyl oder Ethyl steht;

$R^{III}$ für Allyl, Propargyl, für Halogenalkyl mit 1 oder 2 Kohlenstoffatomen und 1 bis 3 gleichen oder verschiedenen Halogenatomen, wie Fluor-und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanoalkyl; ferner für gegebenefalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei $R^1$ bereits genannten Phenylsubstituenten in Frage kommen; oder für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;

$R^{III}$ steht außerdem auch für Methyl, Ethyl, n-Propyl, i-Propyl, n-Butyl, i-Butyl, sec.-Butyl oder tert.-Butyl, wenn gleichzeitig $R^{II}$ für einen Alkylrest steht; oder

$R^{II}$ und $R^{III}$ gemeinsam mit de Stickstoffatom, an das sie gebunden sind, für einen 5-oder 6-gliedrigen Heterocyclus stehen, der gegebenenfalls Sauerstoff-oder ein Stickstoffatom als weitere Heteroatome enthalten kann, wie z.B. Piperidin, Morpholin, Pyrrolidin und Piperazin, und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl oder Methylethylaminocarbonyl substituiert sein kann.

Die bevorzugten und besonders bevorzugten Definitionen für $R^1$, $R^2$, $R^3$ und X, die bei der Formel (I) gegeben wurden, gelten auch für die Verbindungen der Formel (II).

Verwendet man beispielsweise N-(N-Cyanacetyl-glycyl)-benzylamin als Ausgangsstoff und Isoamylnitrit als Derivat der salpetrigen Säure, so kann der Reaktionsablauf des erfindungsgemäßen Verfahrens durch das folgende Formelschema wiedergegeben werden:

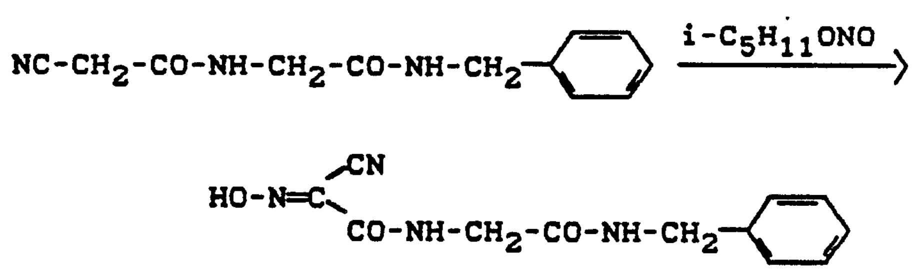


Die für die Durchführung des erfindungsgemäßen Verfahrens als Ausgangsstoffe benötigten Verbindungen sind durch die Formel (II) allgemein definiert. In dieser Formel stehen $R^1$, $R^2$, $R^3$ und X vorzugsweise für diejenigen Bedeutungen, die bereits im Zusammenhang mit der Beschreibung der erfindungsgemäßen Stoffe der Formel (I) für die Reste $R^1$, $R^2$, $R^3$ und X aufgeführt wurden.

Die Verbindungen der Formel (II) sind neu.

Sie können in allgemein bekannter Art und Weise erhalten werden, indem man z.B.

α) Cyanessigester der Formel (III)

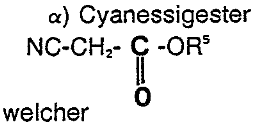 (III)

in welcher
$R^5$ für Methyl oder Ethyl steht,
mit Aminosäurederivaten der Formel (IV)

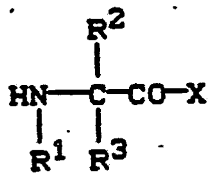 (IV)

in welcher
$R^1$, $R^2$, $R^3$ und X die oben angegebene Bedeutung haben,
gegebenenfalls in einem Lösungsmittel, wie beispielsweise Methanol, Ethanol, Diethylether, 1,2-Dimethoxyethan, Dimethylformamid oder deren Mischungen mit Wasser bei 0°C bis 60°C, vorzugsweise bei Raumtemperatur umsetzt;
oder

β) ein aktiviertes Derivat der Cyanessigsäure der Formel (V)

$NC-CH_2-CO-OH$ (V)

mit Aminosäurederivaten der Formel (IV)

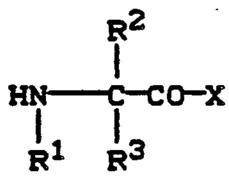 (IV)

in welcher
$R^1$, $R^2$, $R^3$ und X die oben angegebene Bedeutung haben,
in Gegenwart eines organischen Lösungsmittels, wie Tetrahydrofuran oder Dimethylformamid, und gegebenenfalls in Gegenwart eines Säurebindemittels, wie beispielsweise Pyridin oder Triethylamin bei Temperaturen zwischen -40°C und +40°C umsetzt;
als aktivierte Derivate der Cyanessigsäure der Formel (V) werden vorzugsweise das Säurechlorid, aktivierte Ester und gemischte Anhydride eingesetzt;
oder

γ) Halogenacetamide der Formel (VI)

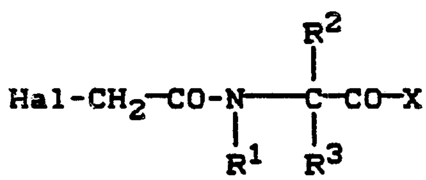 (VI)

in welcher
$R^1$, $R^2$, $R^3$ und X die oben angegebene Bedeutung haben und
Hal für ein Halogenatom, wie Chlor oder Brom steht,
mit Alkalicyaniden der Formel (VII)

MCN (VII)

in welcher
M für ein Alkalimetalläquivalent, wie Natrium oder Kalium steht, in Gegenwart eines Lösungsmittels, wie beispielsweise Wasser, Dimethylsulfoxid, Dimethylformamid, N-Methylpyrrolidon, Acetonitril oder Tetrahydrofuran und gegebenenfalls in Gegenwart eines Phasentransferkatalysators, wie z.B. 18-Krone-6, bei Temperaturen zwischen 0°C und 200°C umsetzt.

Die Verbindungen der Formeln (III), (IV), (V), (VI) und (VII) sind bekannt, bzw. können sie in allgemein bekannter Art und Weise erhalten werden.

Als Verdünnungsmittel kommen für das erfindungsgemäße Verfahren Wasser und organische Lösungsmittel in Frage. Hierzu gehören vorzugsweise Alkohole, wie Methanol oder Ethanol, Carbonsäuren, wie Eisessig sowie Ether, wie Dioxan oder Tetrahydrofuran.

Das erfindungsgemäße Verfahren kann gegebenenfalls im Zweiphasensystem mit Wasser und einem mit Wasser nicht mischbaren Lösungsmittel, wie z.B. Toluol oder Methylenchlorid, durchgeführt werden.

Das erfindungsgemäße Verfahren wird in Gegenwart von salpetriger Säure oder Derivaten der salpetrigen Säure durchgeführt. Man kann alle üblicherweise verwendbaren Derivate der salpetrigen Säure einsetzen. Vozugsweise arbeitet man mit Nitrosylchlorid, Salpetrigsäureanhydrid oder Alkylnitriten, wie Ethylnitrit, tert.-Butylnitrit sowie insbesondere Isoamylnitrit. Die salpetrige Säure wird 'in situ' aus einem Alkalinitrit, wie insbesondere dem Natriumnitrit und einer Säure, wie insbesondere der Salzsäure oder Essigsäure hergestellt.

Das erfindungsgemäße Verfahren wird gegebenenfalls in Gegenwart einer Säure oder Base durchgeführt.

Als Säuren kommen alle üblicherweise verwendbaren organischen und anorganischen Säuren in Frage. Hierzu gehören vorzugsweise Chlorwasserstoff-und Essigsäure.

Als Basen kommen alle üblicherweise verwendbaren starken anorganischen und organischen Basen in Frage. Hierzu gehören vorzugsweise Alkalimetallalkoholate, wie Natriummethylat und Natriumethylat, sowie Alkaliamide, wie Natriumamid.

Die Reaktionstemperaturen können bei der Durchführung des erfindungsgemäßen Verfahrens in einem größeren Bereich variiert werden. Im allgemeinen arbeitet man bei Temperaturen zwischen -20°C und 120°C, vorzugsweise bei Temperaturen zwischen 0°C und 40°C.

Bei der Durchführung des erfindungsgemäßen Verfahrens setzt man auf 1 Mol der Verbindung der Formel (II) im allgemeinen 1 bis 5 Mol, vorzugsweise 1 bis 3 Mol salpetrige Säure bzw. eines Derivates der salpetrigen Säure und ge gebenenfalls 1 bis 5 Mol, vorzugsweise 1 bis 3 Mol Säure oder gegebenenfalls 1 bis 5 Mol, vorzugsweise 1 bis 3 Mol Base ein.

Die Aufarbeitung und Isolierung der Reaktionsprodukte der Formel (I) erfolgt in üblicher Art und Weise, z. B. durch Abfiltrieren von kristallinen Produkten oder durch Extraktion mit einem geeigneten organischen Lösungsmittel.

Die erfindungsgemäßen 2-Cyan-2-oximino-acetamide der Formel (I) eignen sich als Zwischenprodukte zur Synthese von N-(2-Cyan-2-alkoximinoacetyl)-amino-säurederivaten, welche z.B. fungizide Wirksamkeit besitzen.

So lassen sich z.B. N-(2-Cyan-2-alkoximinoacetyl)-aminosäurederivate der Formel (VIII)

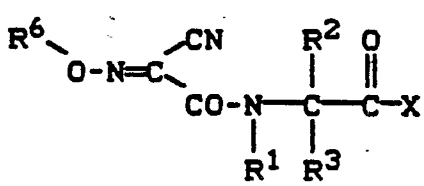

(VIII)

in welcher

$R^1$, $R^2$, $R^3$ und X die oben angegebene Bedeutung haben, und

$R^6$ für Alkyl, Alkenyl, Alkinyl, Cyanalkyl, Azolylalkyl, gegebenenfalls substituiertes Phenylalkyl sowie gegebenenfalls substituiertes Cycloalkyl steht,

herstellen, indem man die erfindungsgemäßen 2-Cyan-2-oximino-acetamide der Formel (I)

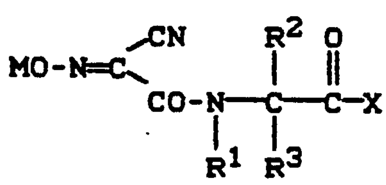

(I)

in welcher

M, $R^1$, $R^2$, $R^3$ und X die oben angegebene Bedeutung haben,

mit Verbindungen der Formel (IX)

$R^6$-W (IX)

in welcher

$R^6$ die oben angegebene Bedeutung hat und

W für Halogen oder einen Sulfonyloxy-Rest steht,

gegebenenfalls in Gegenwart einer Base und gegebenenfalls in Gegenwart eines Lösungsmittels umsetzt.

Als Lösungsmittels kommen für das oben angegebene Verfahren zur Herstellung der N-(2-Cyan-2-alkoximinoacetyl)-amino-säurederivate der Formel (VIII) organische Lösungsmittel in Frage. Hierzu gehören Alkohole, wie Methanol und Ethanol, Ketone, wie Aceton und Methylisobutylketon, Nitrile, wie Acetonitril, Ester, wie Essigester, Amide, wie Dimethylformamid und N-Methylpyrrolidon, sowie Dimethylsulfoxid.

Als Basen kommen für das Verfahren zur Herstellung der N-(2-Cyan-2-alkoximinoacetyl)-aminosäurederivate der Formel (VIII) übliche anorganische und organische Basen in Frage. Hierzu gehören vorzugsweise tertiäre Amine, wie Triethylamin, Pyridin und Diazabicycloundecen (DBU); sowie anorganische Basen, wie Natrium-, Kalium-oder Calciumcarbonat.

Die Reaktionstemperaturen können bei dem oben angegebenen Verfahren zur Herstellung der N-(2-Cyan-2-alkoximinoacetyl)-aminosäurederivate der Formel (VIII) in einem größeren Bereich variiert werden. Im allgemeinen arbeitet man bei Temperaturen zwischen 0°C und 150°C, vorzugsweise bei Temperaturen zwischen 20°C und 120°C.

Bei der Durchführung des oben angegebenen Verfahrens zur Herstellung der N-(2-Cyan-2-alkoximinoacetyl)-aminosäurederivate der Formel (VIII) setzt man pro Mol 2-Cyan-2-oximino-acetamid der Formel (I) im allgemeinen 1 bis 3 Mol, vorzugsweise 1 bis 2 Mol·Alkylierungsmittel der Formel (IX) ein. Die Reaktionsführung, Aufarbeitung und Isolierung der Reaktionsprodukte der Formel (VII) erfolgt in allgemein üblicher Weise.

Bei dem oben angegebenen Verfahren zur Herstellung der N-(2-Cyan-2-alkoximinoacetyl)-aminosäurederivate der Formel (VIII) kann man auch so vorgehen, daß man die 2-Cyan-2-oximino-acetamide der Formel (I) nach dem erfindungsgemäßen Verfahren herstellt und sie ohne Isolierung mit den Alkylierungsmitteln der Formel (IX) wie oben angegeben umsetzt.

Herstellungsbeispiele

Beispiel 1

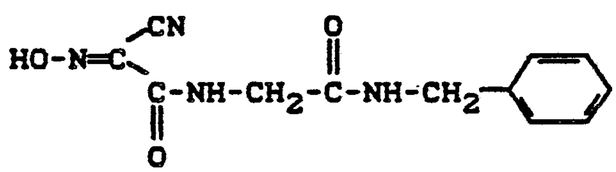

(I-1)

Zu einer Mischung aus 14,8 g (0,064 Mol) N-Cyanacetylglycyl)-benzylamin, 30 ml Methanol und 7,5 g (0,064 Mol) Isoamylnitrit tropft man bei 20°C unter Kühlung 13,6 g (0,065 Mol) einer 26%-igen Lösung von Natriummethylat in Methanol und rührt die Mischung über Nacht (15 Stunden) bei Raumtemperatur. Mit 20%-iger Salzsäure wird angesäuert (pH 2) und im Vakuum weitgehend eingeengt. Man saugt den Niederschlag ab, wäscht ihn mit Toluol und trocknet ihn im Vakuum bei 50°C.

Man erhält 15,9 g (95 % der Theorie) N-N-[(E)-2-Cyan-2-hydroximino-acetyl]-glycyl}-benzylamin vom Schmelzpunkt 182-185°C

Herstellung des Ausgangsproduktes

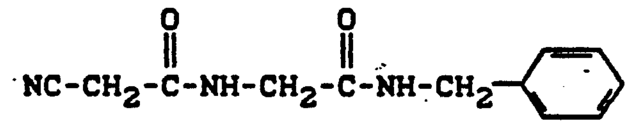

(II-1)

Zu einer Lösung von 9,9 g (0,1 Mol) Cyanessigsäuremethylester und 25,0 g (0,1 Mol) N-Glycyl-benzylamin-hydrobromid in 100 ml Methanol tropft man bei 20°C 20,8 g (0,1 Mol) einer 26%-igen Lösung von Natriummethylat in Methanol und rührt die Mischung über Nacht (15 Stunden). Das Methanol wird im Vakuum abdestilliert, der Rückstand mit 50 ml Wasser verrührt, abgesaugt, mit Ether gewaschen und getrocknet.
Man erhält 16,5 g (71% der Theorie) N-(N-Cyanacetylglycyl)-benzylamin vom Schmelzpunkt129-130°C.

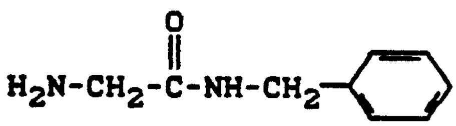

x HBr

Man übergießt 22,4 g (0,075 Mol) N-(N-Benzyloxycarbonylglycyl)-benzylamin mit 82 ml einer 33%-igen Lösung von Bromwasserstoff in Eisessig und rührt die Suspension 2 Stunden bei Raumtemperatur. Das Reaktionsgemisch wird dann mit 140 ml Ether versetzt, der Niederschlag abgesaugt, schnell mit Ether gewaschen und im Vakuum über Kaliumhydroxid getrocknet.
Man erhält 18,0 g (98% der Theorie) N-Glycyl-benzylaminhydrobromid als farblose Kristalle vom Schmelzpunkt 187-188°C.

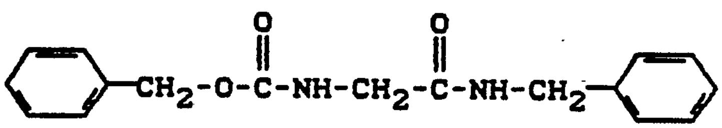

Zu einer Lösung von 250 g (1,195 Mol) N-Benzyloxycarbonylglycin und 120,7 g (1,195 Mol) Triethylamin in 1500 ml Tetrahydrofuran tropft man bei -50°C 165,6 g (1,195 Mol) Chlorameisensäureisobutylester,rührt 10 Minuten bei -50°C und läßt dann möglichst schnell eine vorgekühlte Lösung von 140,8 g (1,315 Mol) Benzylamin in 200 ml Tetrahydrofuran zulaufen, wobei die Temperatur -15°C nicht überschreiten darf. Man rührt 30 Minuten bei -15°C und 15 Stunden bei Raumtemperatur. Das Reaktionsgemisch wird filtriert, das Filtrat eingedampft, der Rückstand in 3000 ml Dichlormethan gelöst, und die Lösung mit Wasser, 1-molarer Salzsäure und gesättigter Natriumhydrogencarbonatlösung gewaschen, getrocknet und eingedampft. Der Rückstand wird durch Verrühren mit 300 ml Petrolether kristallisiert. Man erhält 169g (47% der Theorie) N-(N-Benzyloxycarbonylglycyl)-benzylamin vom Schmelzpunkt 114-117°C.

In analoger Weise und entsprechend dem erfindungsgemäßen Verfahren werden die nachfolgend aufgeführten Verbindungen der Formel (I)

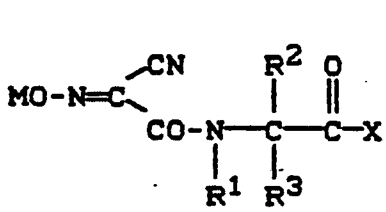

(I)

erhalten:

| Bsp. Nr. | M | $R^1$ | $CR^2R^3$ | X | physikal. Konstante |
|---|---|---|---|---|---|
| I-2 | Na | H | $-CH_2-$ | $-N(C_2H_5)_2$ | Fp:85-90° C |
| I-3 | H | H | $-CH_2-$ | $-N(C_2H_5)_2$ | Fp:157-60° C |

Analog dem Herstellungsbeispiel werden die nachfolgend aufgeführten Verbindungen der Formel (II)

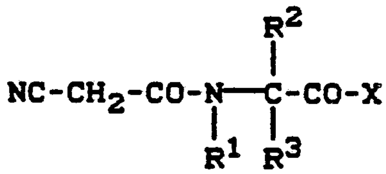

(II)

erhalten:

| Bsp. Nr. | $R^1$ | $CR^2R^3$ | X | physikal. Konstante |
|---|---|---|---|---|
| II-2 | H | $CH_2$ | -OH | Fp:137-40° C |
| II-3 | H | $CH_2$ | $-N(C_2H_5)_2$ | Fp:84-87° C |
| II-4 | H | $CH_2$ | 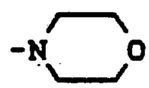 | Fp:148-51° C |
| II-5 | H | $CH_2$ | 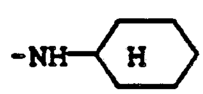 | Fp:181-82° C |

Herstellung eines Folgeproduktes

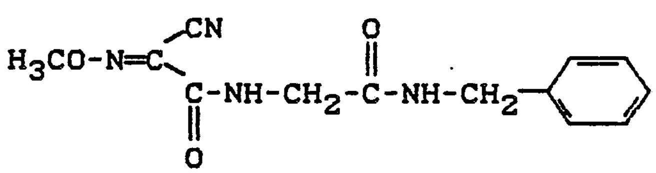

Zu einer Suspension von 90,4 g (0,35 Mol) N-{N-[(E)-2-Cyan-2-hydroximino-acetyl]-glycyl}-benzylamin in 250 ml Aceton gibt man portionsweise 48,3 g (0,35 Mol) pulverisiertes Kaliumcarbonat (Kohlendioxid-Entwicklung) und rührt 30 Minuten bei Raumtemperatur. Dann tropft man 45,1 g (0,39 Mol) Dimethylsulfat so zu, daß die Temperatur 40°C nicht überschreitet. Nach Abklingen der exothermen Reaktion wird 4 Stunden bei Raumtemperatur gerührt. Der Niederschlag wird abgesaugt, mit 50 ml Aceton gewaschen und das Filtrat eingedampft. Der Rückstand wird in 200 ml Dichlormethan gelöst und die Lösung mit je 50 ml gesättigter Natriumhydrogencarbonat-Lösung, Wasser und 1-molarer Salzsäure gewaschen, über Natriumsulfat getrocknet und eingedampft. Der Rückstand wird mit 50 ml Essigester verrührt, abgesaugt und

getrocknet.
Man erhält 51,5 g (54% der Theorie) N-{N-[(E)-2-Cyan-2-methoximino-acetyl]-glycyl}-benzylamin vom Schmelzpunkt 102-104°C.

## Ansprüche

1. 2-Cyan-2-oximino-acetamide der allgemeinen Formel (I)

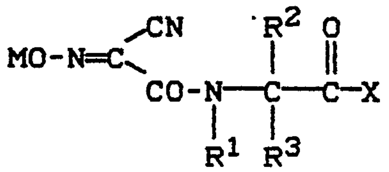

(I)

in welcher
M für Wasserstoff, ein Alkalimetallion, eine protonierte tertiäre Base oder ein quaternäres Ammoniumion steht;
$R^1$ für Wasserstoff, Alkyl, oder jeweils gegebenenfalls substituiertes Phenyl oder Benzyl steht;
$R^2$ für Wasserstoff oder Alkyl steht;
$R^3$ für Wasserstoff, Alkyl, Alkoxycarbonylalkyl, Hydroxycarbonylalkyl, Aminocarbonylalkyl, Azolylalkyl, Cyanalkyl, Hydroxyalkyl, Alkenyl, Alkinyl, gegebenenfalls substituiertes Cycloalkyl, für gegebenenfalls substituiertes Phenyl oder Phenylalkyl oder für die Gruppierung
$R^4SO-_n$-Z-steht, wobei
$R^4$ für Wasserstoff, Alkyl oder gegebenenfalls substituiertes Phenylalkyl steht;
n für die Zahlen 0, 1 oder 2 steht und
Z für eine geradkettige oder verzweigte Alkylenkette steht, oder
$R^1$ und $R^3$ gemeinsam mit dem Stickstoff-Atom und dem Kohlenstoffatom, an die sie gebunden sind, für einen 5-oder 6-gliedrigen Heterocyclus stehen, oder
$R^2$ und $R^3$ gemeinsam mit dem Kohlenstoffatom, an das sie gebunden sind, für Cycloalkyliden stehen und
X für die Gruppierung $-OR^I$ oder $-NR^{II}R^{III}$ steht, wobei
$R^I$ für Wasserstoff, Alkenyl oder für Alkinyl steht;
$R^{II}$ für Wasserstoff oder Alkyl steht;
$R^{III}$ für Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituertes Phenyl oder gegebenenfalls substituiertes Cycloalkyl steht; oder außerdem auch für Alkyl steht, wenn gleichzeitig $R^{II}$ für Alkyl steht,
oder
$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann, und die optischen und geometrischen Isomeren oder die jeweiligen Mischungen.

2. 2-Cyan-oximino-acetamide gemäß Anspruch 1, wobei in der Formel (I)
M für Wasserstoff oder für ein Kalium-oder Natriumäquivalent steht,
$R^1$ für Wasserstoff, geradkettiges oder verzweigtes Alkyl mit 1 bis 4 Kohlenstoffatomen, für jeweils gegebenenfalls einfach bis dreifach, gleich oder verschieden substituiertes Phenyl oder Benzyl steht, wobei als Substituenten vorzugsweise genannt seien: Halogen, Cyano, Nitro, Hydroxy; ferner Alkyl, Alkoxy, Alkylthio, Alkylsulfinyl oder Alkylsulfonyl mit jeweils 1 bis 4 Kohlenstoffatomen und gegebenenfalls einfach bis dreifach, gleich oder verschieden durch Halogen substituiertes Phenyl;
$R^2$ für Wasserstoff oder geradkettiges oder verzweigtes Alkyl mit 1 bis 4 Kohlenstoffatomen steht;
$R^3$ für Wasserstoff, geradkettiges oder verzweigtes Alkyl mit 1 bis 6 Kohlenstoffatomen, für geradkettiges oder verzweigtes Hydroxyalkyl mit 1 bis 4 Kohlenstoffatome, für Alkoxycarbonylalkyl mit jeweils 1 bis 4 Kohlenstoffatomen im Alkoxyteil und im Alkylteil, für Hydroxycarbonylalkyl oder Aminocarbonylalkyl mit jeweils 1 bis 4 Kohlenstoffatomen im Alkylteil, für 1,2,4-Triazol-1-ylalkyl, 1,2,4-Triazol-4-ylalkyl, Imidazol-4-ylalkyl oder Pyrazol-1-ylalkyl mit jeweils 1 bis 6 Kohlenstoffatomen im Alkylteil, für Cyanalkyl mit 1 bis 4 Kohlenstoffatomen im Alkylteil, für Alkenyl oder Alkinyl mit jeweils 2 bis 4 Kohlenstoffatomen, für gegebenefalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt

seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils vorzugsweise die bei $R^1$ bereits genannten Phenylsubstituenten in Frage kommen,
oder für die Gruppierung

$R^4$-$SO_n$-Z-steht, wobei

$R^4$ für Wasserstoff, geradkettiges oder verzweigtes Alkyl mit 1 bis 4 Kohlenstoff atomen, für gegebenenfalls im Phenylteil einfach bis dreifach, gleich oder verschieden substituiertes Phenylalkyl mit 1 bis 4 Kohlenstoffatomen im Alkylteil steht, wobei als Sustituenten die bei $R^1$ bereits genannten Phenylsubstituenten in Frage kommen,

n für die Zahlen 0, 1 oder 2 steht und

Z für eine geradkettige oder verzweigte Alkylenkette mit 1 bis 4 Kohlenstoffatomen steht, oder

$R^1$ und $R^3$ gemeinsam mit dem Stickstoffatom und dem Kohlenstoffatom, an die sie gebunden sind, für eine 5-oder 6-gliedrigen Heterocyclus stehen, oder

$R^2$ und $R^3$ gemeinsam mit dem Kohlenstoffatom, an das sie gebunden sind, für Cycloalkyliden mit 3 bis 6 Kohlenstoffatomen stehen;

X für die Gruppierungen -$OR^I$ oder -$NR^{II}R^{III}$steht, wobei

$R^I$ für Wasserstoff, Alkenyl oder Alkinyl mit jeweils 2 bis 4 Kohlenstoffatomen steht;

$R^{II}$ für Wasserstoff oder geradkettiges oder verzweigtes Alkyl mit 1 bis 4 Kohlenstoffatomen steht;

$R^{III}$ für Alkenyl oder Alkinyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit 1 bis 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei $R^1$ bereits genannten Phenylsubstituenten in Frage kommen, für gegebenenfalls gleich oder verschieden substituiertes Cycloalkyl mit 3 bis 7 Kohlenstoffatomen, wobei als Substituenten vorzugsweise Halogen und Alkyl mit 1 bis 4 Kohlenstoffatomen in Frage kommen; $R^{III}$ steht auch für geradkettiges oder verzweigtes Alkyl mit 1 bis 4 Kohlen stoffatomen, wenn $R^{II}$ gleichzeitig für Alkyl steht;

oder

$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff oder Stickstoff als weitere Heteratome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl, Alkylaminocarbonyl oder Dialkylaminocarbonyl mit jeweils 1 bis 4 Kohlenstoffatomen in den einzelnen Alkylteilen substituiert sein kann, und die optischen und geometrischen Isomeren oder die jeweiligen Mischungen.

3. 2-Cyano-2-oximino-acetamide gemäß Anspruch 1, wobei in der Formel (I)

M für Wasserstoff, für ein Kalium-oder Natriumäquivalent steht,

$R^1$ für Wasserstoff, Methyl, Ethyl, für jeweils gegebenenfalls einfach oder zweifach, gleich oder verschiedenen substituiertes Phenyl oder Benzyl steht, wobei als Substituenten insbesondere genannt seien: Fluor, Chlor, Cyano, Nitro, Hydroxy, Methyl, Methoxy, Methylthio, Methyl sulfinyl, Methylsulfonyl und gegebenenfalls einfach oder zweifach, gleich oder verschieden durch Fluor oder Chlor substituiertes Phenyl steht;

$R^2$ für Wasserstoff, Methyl oder Ethyl steht;

$R^3$ für Wasserstoff, geradkettiges oder verzweigtes Alkyl mit 1 bis 4 Kohlenstoffatomen, Hydroxymethyl, 1-Hydroxyethyl, für Methoxycarbonylmethyl, Methoxycarbonylethyl, Ethoxycarbonylmethyl, Ethoxycarbonylethyl, Hydroxycarbonylmethyl, Hydroxycarbonylethyl, Aminocarbonylmethyl, Aminocarbonylethyl, für 1,2,4-Triazol-1-ylalkyl, 1,2,4-Triazol-4-ylalkyl, Imidazol-4-ylalkyl und Pyrazol-1-ylalkyl mit jeweils 1 bis 4 Kohlenstoffatomen im Alkylteil, für Cyanmethyl, Cyanethyl, für Allyl, für Propargyl, für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht; ferner für gegebenenfalls im Phenylteil einfach oder zweifach, gleich oder verschieden substitiertes Phenylalkyl mit 1 oder 2 Kohlenstoffatomen im Alkylteil oder einfach oder zweifach, gleich oder verschieden substituertes Phenyl steht, wobei als Substituenten die bei $R^1$ bereits genannten Phenylsubstituenten in Frage kommen, ferner für die Gruppierung

$R^4$-$SO_n$-Z-steht, wobei

$R^4$ für Wasserstoff, Methyl, Ethyl oder für gegebenenfalls im Phenylteil einfach oder zweifach, gleich oder

verschieden substituiertes Phenylalkyl mit 1 oder 2 Kohlenstoffatomen im Alkylteil steht, wobei als Substituenten insbesondere die bei $R^1$ bereits genannten Phenylsubstituenten in Frage kommen,
n für die Zahlen 0, 1 oder 2 steht und
Z für eine geradkettige oder verzweigte Alkylenkette mit 1 oder 2 Kohlenstoffatomen steht, oder
$R^1$ und $R^2$ gemeinsam mit dem Stickstoffatom und dem Kohlenstoffatom, an die sie gebunden sind, einen Piperidin-oder Pyrrolidinring bilden, oder
$R^2$ und $R^3$ geminsam mit dem Kohlenstoffatom, an das sie gebunden sind, für Cyclopropyliden stehen, und
X für die Gruppierung -$OR^I$ oder -$NR^{II}R^{III}$ steht, wobei
$R^I$ für Wasserstoff, Allyl oder Propargyl steht;
$R^{II}$ für Wasserstoff, Methyl oder Ethyl steht;
$R^{III}$ für Allyl, Propargyl, für Halogenalkyl mit 1 oder 2 Kohlenstoffatomen und 1 bis 3 gleichen oder verschiedenen Halogenatomen, wie Fluor-und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanoalkyl; ferner für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei $R^1$ bereits genannten Phenylsubstituenten in Frage kommen; oder für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;
$R^{III}$ steht außerdem auch für Methyl, Ethyl, n-Propyl, i-Propyl, n-Butyl, i-Butyl, sec.-Butyl oder tert.-Butyl, wenn gleichzeitig $R^{II}$ für einen Alkylrest steht; oder
$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff-oder ein Stickstoffatom als weitere Heteroatome enthalten kann, und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann, und die optischen und geometrischen Isomeren und die jeweiligen Mischungen.

4. Verfahren zur Herstellung von 2-Cyan-2-oximino-acetamiden der allgemeinen Formel (I)

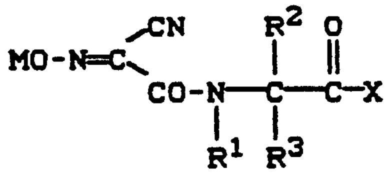

(I)

in welcher
M für Wasserstoff, ein Alkalimetallion, eine protonerte tertiäre Base oder ein quaternäres Ammoniumion steht;
$R^1$ für Wasserstoff, Alkyl, oder jeweils gegebenenfalls substituiertes Phenyl oder Benzyl steht;
$R^2$ für Wasserstoff oder Alkyl steht;
$R^3$ für Wasserstoff, Alkyl, Alkoxycarbonylalkyl, Hydroxycarbonylalkyl, Aminocarbonylalkyl, Azolylalkyl, Cyanalkyl, Hydroxyalkyl, Alkenyl, Alkinyl, gegebenenfalls substituiertes Cycloalkyl, für gegebenenfalls substituiertes Phenyl oder Phenylalkyl oder für die Gruppierung
$R^4$-$SO_n$-Z-steht, wobei
$R^4$ für Wasserstoff, Alkyl oder gegebenenfalls substituiertes Phenylalkyl steht;
n für die Zahlen 0, 1 oder 2 steht und
Z für eine geradkettige oder verzweigte Alkylenkette steht, oder
$R^1$ und $R^3$ gemeinsam mit dem Stickstoff-Atom und dem Kohlenstoffatom, an die sie gebunden sind, für einen 5-oder 6-gliedrigen Heterocyclus stehen, oder
$R^2$ und $R^3$ gemeinsam mit dem Kohlenstoffatom, an das sie gebunden sind, für Cycloalkyliden stehen und
X für die Gruppierung -$OR^I$ oder -$NR^{II}R^{III}$ steht, wobei
$R^I$ für Wasserstoff, Alkenyl oder für Alkinyl steht;
$R^{II}$ für Wasserstoff oder Alkyl steht;
$R^{III}$ für Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl oder gegebenenfalls substituiertes Cycloalkyl steht; oder außerdem auch für Alkyl steht, wenn gleichzeitig $R^{II}$ für Alkyl steht,
oder
$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann, deren optische und geoemetrische Isomere oder die jeweiligen Mischungen,
dadurch gekennzeichnet, daß man Verbindungen der Formel (II)

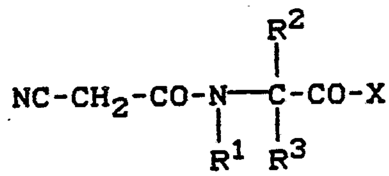
(II)

in welcher
$R^1$, $R^2$, $R^3$ und X die oben angegebene Bedeutung haben,
mit salpetriger Säure oder Derivaten der salpetrigen Säure gegebenenfalls in Gegenwart eines Verdünnungsmittels und gegebenenfalls in Gegenwart einer Säure oder einer Base umsetzt.

5. Verwendung von 2-Cyan-2-oximino-acetamiden der Formel (I) gemäß den Ansprüchen 1 und 4 als Ausgangsstoffe zur Herstellung hochaktiver Verbindungen.

6. Verbindungen der Formel (II)

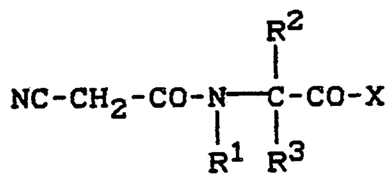
(II)

in welcher
$R^1$ für Wasserstoff, Alkyl, oder jeweils gegebenenfalls substituiertes Phenyl oder Benzyl steht;
$R^2$ für Wasserstoff oder Alkyl steht;
$R^3$ für Wasserstoff, Alkyl, Alkoxycarbonylalkyl, Hydroxycarbonylalkyl, Aminocarbonylalkyl, Azolylalkyl, Cyanalkyl, Hydroxyalkyl, Alkenyl, Alkinyl, gegebenenfalls substituiertes Cycloalkyl, für gegebenenfalls substituiertes Phenyl oder Phenylalkyl oder für die Gruppierung
$R^4$-$SO_n$-Z-steht, wobei
$R^4$ für Wasserstoff, Alkyl oder gegebenenfalls substituiertes Phenylalkyl steht;
n für die Zahlen 0, 1 oder 2 steht und
Z für eine geradkettige oder verzweigte Alkylenkette steht, oder
$R^1$ und $R^3$ gemeinsam mit dem Stickstoff-Atom und dem Kohlenstoffatom, an die sie gebunden sind, für einen 5-oder 6-gliedrigen Heterocyclus stehen, oder
$R^2$ und $R^3$ gemeinsam mit dem Kohlenstoffatom, an das sie gebunden sind, für Cycloalkyliden stehen und
X für die Gruppierung -$OR^I$ oder -$NR^{II}R^{III}$ steht, wobei
$R^I$ für Wasserstoff, Alkenyl oder für Alkinyl steht;
$R^{II}$ für Wasserstoff oder Alkyl steht;
$R^{III}$ für Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl oder gegebenenfalls substituiertes Cycloalkyl steht; oder außerdem auch für Alkyl steht, wenn gleichzeitig $R^{II}$ für Alkyl steht,
oder
$R^{II}$ und $R^{III}$ gemeinsam mit dem Stickstoff-Atom, an das sie gebunden sind, für einen gegebenen falls substituierten Heterocyclus stehen, der weitere Heteroatome enthalten kann.

7. Verbindungen gemäß Anspruch 6, wobei in der Formel (II)
$R^1$ für Wasserstoff, geradkettiges oder verzweigtes Alkyl mit 1 bis 4 Kohlenstoffatomen, für jeweils gegebenenfalls einfach bis dreifach, gleich oder verschieden substituiertes Phenyl oder Benzyl steht, wobei als Substituenten vorzugsweise genannt seien: Halogen, Cyano, Nitro, Hydroxy; ferner Alkyl, Alkoxy, Alkylthio, Alkylsulfinyl oder Alkylsulfonyl mit jeweils 1 bis 4 Kohlenstoffatomen und gegebenenfalls einfach bis dreifach, gleich oder verschieden durch Halogen substituiertes Phenyl;

$R^2$ für Wasserstoff oder geradkettiges oder verzweigtes Alkyl mit 1 bis 4 Kohlenstoffatomen steht;

$R^3$ für Wasserstoff, geradkettiges oder verzweigtes Alkyl mit 1 bis 6 Kohlenstoffatomen, für geradkettiges oder verzweigtes Hydroxyalkyl mit 1 bis 4 Kohlenstoffatomen, für Alkoxycarbonylalkyl mit jeweils 1 bis 4 Kohlenstoffatomen im Alkoxyteil und im Alkylteil, für Hydroxycarbonylalkyl oder Aminocarbonylalkyl mit jeweils 1 bis 4 Kohlenstoffatomen im Alkylteil, für 1,2,4-Triazol-1-ylalkyl, 1,2,4-Triazol-4-ylalkyl, Imidazol-4-ylalkyl und Pyrazol-1-ylalkyl mit jeweils 1 bis 6 Kohlenstoffatomen im Alkylteil, für Cyanalkyl mit 1 bis 4 Kohlenstoffatomen in Alkylteil, für Alkenyl oder Alkinyl mit jeweils 2 bis 4 Kohlenstoffatomen, für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien, ferner für gegebenenfalls im Phenylteil einfach bis dreifach, gleich oder verschieden substituiertes Phenyl oder Phenylalkyl mit 1 bis 4 Kohlenstoffatomen im Alkylteil steht, wobei als Substituenten jeweils vorzugsweise die bei $R^1$ bereits genannten Phenylsubstituenten in Frage kommen,

oder für die Gruppierung

$R^4$-$SO_n$-Z-steht, wobei

$R^4$ für Wasserstoff, geradkettiges oder verzweigtes Alkyl mit 1 bis 4 Kohlenstoffatomen, für gegebenenfalls im Phenylteil einfach bis dreifach, gleich oder verschieden substituiertes Phenylalkyl mit 1 bis 4 Kohlenstoffatomen im Alkylteil steht, wobei als Substituenten die bei $R^1$ bereits genannten Phenylsubstituenten in Frage kommen,

n für die Zahlen 0, 1 oder 2 steht und

Z für eine geradkettige oder verzweigte Alkylenkette mit 1 bis 4 Kohlenstoffatomen steht, oder

$R^1$ und $R^3$ gemeinsam mit dem Stickstoffatom und dem Kohlenstoffatom, an die sie gebunden sind, für einen 5-oder 6-gliedrigen Heterocyclus stehen oder

$R^2$ und $R^3$ gemeinsam mit dem Kohlenstoffatom, an das sie gebunden sind, für Cycloalkyliden mit 3 bis 6 Kohlenstoffatomen stehen;

X für die Gruppierungen -$OR^I$ oder -$NR^{II}R^{III}$steht, wobei

$R^I$ für Wasserstoff, Alkenyl oder Alkinyl mit jeweils 2 bis 4 Kohlenstoffatomen steht;

$R^{II}$ für Wasserstoff oder geradkettiges oder verzweigtes Alkyl mit 1 bis 4 Kohlenstoffatomen steht;

$R^{III}$ für Alkenyl oder Alkinyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit 1 bis 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei $R^1$ bereits genannten Phenylsubstituenten in Frage kommen, für gegebenenfalls gleich oder verschieden substituiertes Cycloalkyl mit 3 bis 7 Kohlenstoffatomen, wobei als Substituenten vorzugsweise Halogen und Alkyl mit 1 bis 4 Kohlenstoffatomen in Frage kommen; $R^{III}$ steht auch für geradkettiges oder verzweigtes Alkyl mit 1 bis 4 Kohlenstoffatomen, wenn $R^{II}$ gleichzeitig für Alkyl steht;

oder

$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff oder Stickstoff als weitere Heteroatome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl, Alkylaminocarbonyl oder Dialkylaminocarbonyl mit jeweils 1 bis 4 Kohlenstoffatomen in den einzelnen Alkylteilen substituiert sein kann.

8. Verbindungen gemäß Anspruch 6, wobei in der Formel (II)

$R^1$ für Wasserstoff, Methyl, Ethyl, für jeweils gegebenenfalls einfach oder zweifach, gleich oder verschiedenen substituiertes Phenyl oder Benzyl steht, wobei als Substituenten insbesondere genannt seien: Fluor, Chlor, Cyano, Nitro, Hydroxy, Methyl, Methoxy, Methylthio, Methylsulfinyl, Methylsulfonyl oder gegebenenfalls einfach oder zweifach, gleich oder verschieden durch Fluor oder Chlor substituiertes Phenyl steht;

$R^2$ für Wasserstoff, Methyl oder Ethyl steht;

$R^3$ für Wasserstoff, geradkettiges oder verzweigtes Alkyl mit 1 bis 4 Kohlenstoffatomen, Hydroxymethyl, 1-Hydroxyethyl, für Methoxycarbonylmethyl, Methoxycarbonylethyl, Ethoxycarbonylmethyl, Ethoxycarbonylethyl, Hydroxycarbonylmethyl, Hydroxycarbonylethyl, Aminocarbonylmethyl, Aminocarbonylethyl, für 1,2,4-Triazol-1-ylalkyl, 1,2,4-Triazol-4-ylalkyl, Imidazol-4-ylalkyl oder Pyrazol-1-ylalkyl mit jeweils 1 bis 4 Kohlenstoffatomen im Alkylteil, für Cyanmethyl, Cyanethyl, für Allyl, für Propargyl, für jeweils gegebenenfalls

einfach oder zweifach, gleich oder verschieden durch Fluor, Chlor oder Methyl substituiertes Cyclopropyl, Cyclobutyl, Cyclopentyl, Cyclohexyl oder Cycloheptyl steht; ferner für gegebenenfalls im Phenylteil einfach oder zweifach, gleich oder verschieden substituiertes Phenylalkyl mit 1 oder 2 Kohlenstoffatomen in Alkylteil oder einfach oder zweifach, gleich oder verschieden substituiertes Phenyl steht, wobei als Substituenten die bei $R^1$ bereits genannten Phenylsubstituenten in Frage kommen, ferner für die Gruppierung

$R^4$-$SO_n$-Z-steht, wobei

$R^4$ für Wasserstoff, Methyl, Ethyl oder für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere die bei $R^1$ bereits genannten Phenylsubstituenten in Frage kommen,

n für die Zahlen 0, 1 oder 2 steht und

Z für eine geradkettige oder verzweigte Alkylenkette mit 1 oder 2 Kohlenstoffatomen steht, oder

$R^1$ und $R^2$ gemeinsam mit dem Stickstoffatom und dem Kohlenstoffatom, an die sie gebunden sind, einen Piperidin-oder Pyrrolidinring bilden, oder

$R^2$ und $R^3$ gemeinsam mit dem Kohlenstoffatom, an das sie gebunden sind, für Cyclopropyliden stehen, und

X für die Gruppierung -$OR^I$ oder -$NR^{II}$ $R^{III}$ steht, wobei

$R^I$ für Wasserstoff, Allyl oder Propargyl steht;

$R^{II}$ für Wasserstoff, Methyl oder Ethyl steht;

$R^{III}$ für Allyl, Propargyl, für Halogenalkyl mit 1 oder 2 Kohlenstoffatomen und 1 bis 3 gleichen oder verschiedenen Halogenatomen, wie Fluor-und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanoalkyl; ferner für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei $R^1$ bereits gennanten Phenylsubstituenten in Frage kommen; oder für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;

$R^{III}$ steht außerdem auch für Methyl, Ethyl, n-Propyl, i-Propyl, n-Butyl, i-Butyl, sec.-Butyl oder tert.-Butyl, wenn gleichzeitig $R^{II}$ für einen Alkylrest steht; oder

$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff-oder ein Stickstoffatom als weitere Heteroatome enthalten kann, und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann.

9. Verfahren zur Herstellung von Verbindungen der Formel (II)

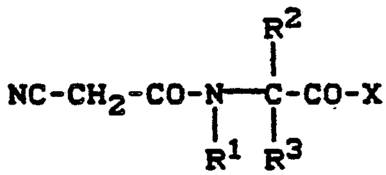

(II)

in welcher

$R^1$ für Wasserstoff, Alkyl, oder jeweils gegebenenfalls substituiertes Phenyl oder Benzyl steht;

$R^2$ für Wasserstoff oder Alkyl steht;

$R^3$ für Wasserstoff, Alkyl, Alkoxycarbonylalkyl, Hydroxycarbonylalkyl, Aminocarbonylalkyl, Azolylalkyl, Cyanalkyl, Hydroxyalkyl, Alkenyl, Alkinyl, gegebenenfalls substituiertes Cycloalkyl, für gegebenenfalls substituiertes Phenyl oder Phenylalkyl oder für die Gruppierung

$R^4$-$SO_n$-Z-steht, wobei

$R^4$ für Wasserstoff, Alkyl oder gegebenenfalls substituiertes Phenylalkyl steht;

n für die Zahlen 0, 1 oder 2 steht und

Z für eine geradkettige oder verzweigte Alkylenkette steht, oder

$R^1$ und $R^3$ gemeinsam mit dem Stickstoff-Atom und dem Kohlenstoffatom, an die sie gebunden sind, für einen 5-oder 6-gliedrigen Heterocyclus stehen, oder

$R^2$ und $R^3$ gemeinsam mit dem Kohlenstoffatom, an das sie gebunden sind, für Cycloalkyliden stehen und

X für die Gruppierung -$OR^I$ oder -$NR^{II}R^{III}$ steht, wobei

$R^I$ für Wasserstoff, Alkenyl oder für Alkinyl steht;
$R^{II}$ für Wasserstoff oder Alkyl steht;
$R^{III}$ für Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl oder gegebenenfalls substituiertes Cycloalkyl steht; oder außerdem auch für Alkyl steht, wenn gleichzeitig $R^{II}$ für Alkyl steht,
oder
$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann, dadurch gekennzeichnet, daß man
α) Cyanessigester der Formel (III)

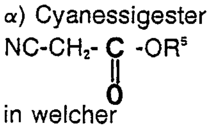 (III)

in welcher
$R^5$ für Methyl oder Ethyl steht,
mit Aminosäurederivaten der Formel (IV)

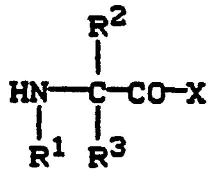

(IV)

in welcher
$R^1$, $R^2$, $R^3$ und X die oben angegebene Bedeutung haben,
gegebenenfalls in einem Lösungsmittel umsetzt;
oder
β) ein aktiviertes Derivat der Cyanessigsäure der Formel (V)

$NC-CH_2-CO-OH$ (V)

mit Aminosäurederivaten der Formel (IV)

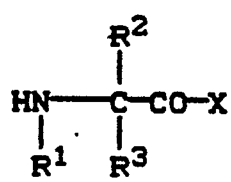

(IV)

in welcher
$R^1$, $R^2$, $R^3$ und X die oben angegebene Bedeutung haben,
in Gegenwart eines organischen Lösungsmittels und gegebenenfalls in Gegenwart eines Säurebindemittels, bei Temperaturen zwischen -40°C und +40°C umsetzt,
oder
γ) Halogenacetamide der Formel (VI)

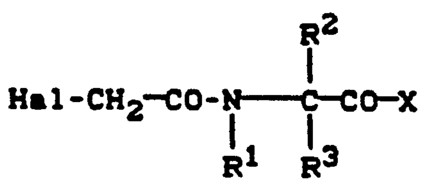

(VI)

in welcher
$R^1$, $R^2$, $R^3$ und X die oben angegebene Bedeutung haben und
Hal für ein Halogenatom steht,
mit Alkalicyaniden der Formel (VII)

MCN (VII)

in welcher
M für ein Alkalimetalläquivalent steht,
in Gegenwart eines Lösungsmittels und gegebenenfalls in Gegenwart eines Phasentransferkatalysators, bei Temperaturen zwischen 0°C und 200°C umsetzt.

Europäisches Patentamt

# EUROPÄISCHER RECHERCHENBERICHT

Nummer der Anmeldung

EP 88 10 0645

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 201 999 (ICI)<br>* Anspruch 1, Tabelle I, Verbindungen 23, 24, 93 *<br>--- | 1 | C 07 C 131/00<br>C 07 C 147/02<br>C 07 C 147/14<br>C 07 C 149/20<br>C 07 C 121/43<br>C 07 D 207/02<br>C 07 D 211/84 |
| A | US-A-3 306 909 (R.H. ULOTH)<br>* Spalte 1, Formel II *<br>--- | 4 | |
| A | EP-A-0 206 004 (BAYER)<br>* * Anspruch 1 * & DE - A - 3 602 243 (Kat. A,D) *<br>----- | 1,5 | |
| | | | **RECHERCHIERTE SACHGEBIETE (Int. Cl.4)** |
| | | | C 07 C 131/00<br>C 07 C 147/02<br>C 07 C 147/14<br>C 07 C 149/20<br>C 07 C 121/43<br>C 07 D 207/02<br>C 07 D 211/04 |

Der vorliegende Recherchenbericht wurde für alle Patentansprüche erstellt

| Recherchenort | Abschlußdatum der Recherche | Prüfer |
|---|---|---|
| BERLIN | 18-04-1988 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)